# EUROPÄISCHE PATENTSCHRIFT

(11) **EP 2 029 094 B1**
(45) Veröffentlichungstag und Bekanntmachung des Hinweises auf die Patenterteilung: **26.10.2011**
(21) Anmeldenummer: 07729258.9
(22) Anmeldetag: 18.05.2007
(51) Int. Cl.: A61K 8/44, A61K 8/365, A61K 8/97, A61Q 19/00

(54) **VERWENDUNG VON ZUBEREITUNGEN MIT EINEM GEHALT AN HYDROXYCITRAT UND CARNITIN ALS WIRKSAME PRINZIPIEN ZUR SEBUMREDUKTION**
USE OF PREPARATIONS CONTAINING HYDROXYCITRATE AND CARNITINE AS ACTIVE PRINCIPLES FOR SEBUM REDUCTION
UTILISATION DE PRÉPARATIONS CONTENANT DE L'ACIDE HYDROXYCITRIQUE ET DE LA CARNITINE COMME PRINCIPES ACTIFS POUR RÉDUIRE LA PRODUCTION DE SÉBUM

(30) Priorität: 22.05.2006 DE 102006024659; 10.07.2006 DE 102006032015
(43) Veröffentlichungstag der Anmeldung: 04.03.2009
(73) Patentinhaber: Beiersdorf AG, 20253 Hamburg (DE)
(72) Erfinder: KRUSE, Inge, 20146 Hamburg (DE); SCHMIDT-ROSE, Thomas, 20251 Hamburg (DE); PEIRANO, Reto Ivo, 22589 Hamburg (DE); WOEHRMANN, Michael, 22525 Hamburg (DE)
(86) Internationale Anmeldenummer: PCT/EP2007/054810
(87) Internationale Veröffentlichungsnummer: WO 2007/135083

(56) Entgegenhaltungen:
- WO-A-02/092029
- ES-A1- 2 106 689
- FR-A1- 2 716 374

## Beschreibung

Die vorliegende Erfindung betrifft die Verwendung von Wirkstoffen und Zubereitungen, solche Wirkstoffe enthaltend, welche zur Verringerung der Sebumproduktion in der Talkdrüse wirksam sind.

Fettige Haut im Sinne der vorliegenden Schrift entsteht durch eine übermäßige Sebumproduktion der Sebozyten der Talgdrüse. Sebum besteht aus einer Mischung von neutralen Lipiden (Triglyceride, Wachsester, Squalen und Cholesterolester), welche durch den Talgdrüsenausführgang auf die Hautoberfläche gelangt, und sich dort mit körpereigenem Schweiß zu einem Hydrolipidfilm vermischt. Eine übermäßige Sebumproduktion führt zu dem Gefühl von fettiger Haut, welche auch als glänzend, schmierig oder unrein empfunden wird. Fettige Haut ist bei unterschiedlichen Altersgruppen und bei Männern und Frauen verbreitet, wobei gerade auch Jugendliche eine verstärkte Sebumproduktion aufweisen, welche eine wesentliche Ursache für unreine Haut und Akne ist. Fettige Haut ist ein kosmetisch unerwünschter Zustand, welcher durch die Anwendung von sebumreduzierenden Wirkstoffen verhindert werden soll.

Fettiges Haar im Sinne der vorliegenden Schrift entsteht durch eine übermäßige Sebumproduktion der Talgdrüsen der Kopfhaut. Das Sebum gelangt dabei auf die Haare der Kopfhaut, wobei der kosmetisch unerwünschte Zustand der fettigen Haare entsteht. Dieser ist gekennzeichnet durch eine kosmetisch unerwünschte Anmutung, einer schlechten Frisierbarkeit und durch wenig Volumen.

Bei der unreinen Haut sowie bei milden Formen der Akne sind neben einer verstärkten Sebumproduktion auch andere Einflüsse wie z. B. bakterielle Sekundärinfektionen von ätiologischer Bedeutung. Einer der wichtigsten Mikroorganismen, der in Zusammenhang mit unreiner Haut steht, ist Propionibacterium acnes.

Unreine Haut und/oder Komedonen beeinträchtigen das Wohlbefinden der Betroffenen aber selbst in leichten Fällen. Da praktisch jeder oder jede Jugendliche von unreiner Haut irgendeiner Ausprägung betroffen ist, besteht bei vielen Personen Bedarf, diesem Zustande abzuhelfen. Da ein wesentlicher Faktor dabei die übermäßige Talgproduktion ist, kann in vielen Fällen bereits durch den Einsatz von sebumreduzierenden Wirkstoffen eine Verbesserung des Hautzustandes erreicht werden.

Aufgabe der vorliegenden Erfindung war es also, zur Verringerung der Sebumpoduktion in der Talgdrüse wirksame Zubereitungen zu finden.

Der Stand der Technik kennt bereits einige Versuche zur Lösung dieses Problems, die jedoch nicht alle Vorteile der hier vorgestellten Entwicklung kennen.

Die Schrift US2004/0208902 (Gupta) offenbart eine Zubereitung mit einer Zeolithzubereitung und einem topischen Begrenzt-Freisetzungs- und Transportsystem für eine hautförderliche Zubereitung.

Die Schrift US 2004/0161435 (Gupta) offenbart eine kosmetische Maske mit hautförderlicher Zubereitung, einer fettreduzierenden Zubereitung und einer Bindungszubereitung.

Die Schrift US 2004/0219124 (Gupta) offenbart eine kosmetische Maske mit bestimmten metallkomplexierenden Zubereitungen, Metallkationen und einer hautförderlichen Zubereitung.

Die Schrift US 2004/146539 (Gupta) offenbart eine synergistische, gegen eine Vielzahl von Leiden wirkende Zubereitung mit einer hautförderlichen Zubereitung, einer weiteren Behandlungszubereitung, einer die Synthese von Kollagen und Elastin fördernden Zubereitung.

Die Schrift WO 03/9854 (Lonza) offenbart eine Zubereitung mit Carnitin oder Acylcarnitin und Phytosterol oder Phytostanol.

Die Schrift WO 99/07388 (Maccarty et al.) offenbart eine Methode zu Gewichtsreduzierung.

Die Schrift US 2004/0185069 (Gupta) offenbart eine Zubereitung u. a. zur Fettreduktion mit u. a. Hydroxycitrat oder deren Derivaten.

Die Schrift WO2004/74216 (VAMA) offenbart Carnitinsalze der Hydroxyzitronensäure.

Die Schrift ES 2106689 offenbart Formulierungen mit Hydroxyzitronensäure, die auch Carnitin enthalten können.

FR 2716374 offenbart Zubereitungen mit Pflanzenextrakten, die auch aus Garcinia cambogia gewonnen sein können.

All diese Dokumente schweigen aber dazu, wie besonders vorteilhaft die Sebumproduktion in der Talgdrüse verringert werden kann.

Es wurde überraschend gefunden, und darin liegt die Lösung der Aufgabe, dass die nichttherapeutische Verwendung von topischen Zubereitungen enthaltend Hydroxycitrat wobei als Hydroxycitrat enthaltende Komponente ein *Garcinia Cambogia-Extrakt* verwendet wird und zusätzlich Carnitin in der topischen Zubereitung enthalten ist und die Zubereitung einen Hydroxycitrat-Anteil von 0.25% bis 2% und einen Carnitin-Anteil von 1% bis 4% aufweist zur Verringerung der Sebumproduktion in der Talgdrüse den Nachteilen des Standes der Technik abhilft. Es kann durch die Anwendung der sebumreduzierenden Wirkstoffe auf der Kopfhautregion die Sebumproduktion gesenkt und somit der kosmetisch unerwünschte Zustand der fettigen Haare verhindert werden. Bevorzugt ist es, wenn Alkali- oder Erdalkali-Hydroxycitrate verwendet werden. Besonders bevorzugt sind wasserlösliche Hydroxycitrate wie beispielsweise Calcium-Kalium- und Kalium-Salze.

Besonders bevorzugt ist es, wenn zusätzlich saure Aluminium- oder Alumuminium-Zirkoniumsalze in der topischen Zubereitung enthalten sind.

Die Sebumlipid-Reduktion durch Carnitin und/oder Hydroxycitrat wurden durch einen Zellkulturtest mit einer humanen Sebozytenzelllinie untersucht. Dabei erfolgte die Messung der neutralen Sebumlipide mittels Nilrot. Verwendet wurde als Wirkstoff ein aufbereiteter *Garcinia* Cambogia Extrakt, der ca. 50-60% Hydroxycitrat als Calcium-Kalium-Mischsalz enthält.

In Prozent angegebene Gehalte in dieser Schrift verstehen sich als Massenprozente.

Der Einfachheit halber wird in dieser Schrift allgemein von Hydroxycitrat oder vom *Garcinia Cambogia*-Extrakt, gesprochen. Gemeint sind damit alle möglichen Formen von Alkali- und Erdalkalisalzen von Hydroxycitrat, wie sie beispielsweise nach Aufbereitung von *Garcinia Cambogia*-Extrakten erhältlich sind. Insbesondere bevorzugt sind die wasserlöslichen Kalium- und Calcium-Kalium-Salze von Hydroxycitrat, wie sie beispielsweise von der Firma Sabinsa unter dem Namen Citrin® oder Citrin CaK® angeboten werden. Im Sinne der Erfindung zu verstehen sind auch rein synthetische Herstellungsverfahren von Hydroxycitrat-Salzen oder Isolierungen aus anderen Pflanzenextrakten. Die Verhältnisse oder in Prozent angegebenen Gehalte von Hydroxycitrat in dieser Schrift beziehen sich, falls nicht expilzit erwähnt, auf den *Garcinia Cambogia* Extrakt, in dem Hydroxycitrat mit ca. 50-60% enthalten ist.

Folgende Ergebnisse wurden erhalten:

| **Sebumreduzierende Wirkstoffe** | **gemessene Reduktion** | **additive Reduktion** |
|---|---|---|
| Kontrolle (Medium) | 0% | |
| 1% Carnitin | 38% | |
| 1,5% Carnitin | 54% | |
| 0,25% *Garcinia Cambogia*-Extrakt | 13% | |
| 0,5% *Garcinia Cambogia*-Extrakt | 46% | |
| 1% *Garcinia Cambogia*-Extrakt | 42% | |
| 1% Carnitin & 0,5% *Garcinia Cambogia*-Extrakt | 68% | 0,334 = 67% |
| 1% Carnitin & 1% *Garcinia Cambogia*-Extrakt | 66% | 0,357 = 64% |
| 1,5% Carnitin & 1% *Garcinia Cambogia*-Extrakt | 88% | 0,264 = 74% |

Aus diesen Daten ist ersichtlich, dass bereits der *Garcinia Cambogia* Extrakt alleine eine gute Sebumreduktion zeigt, die insbesondere durch die Kombination mit Carnitin noch deutlich gesteigert werden kann.

Überraschenderweise und für den Fachmann nicht vorhersehbar, zeigt eine Kombination von dem *Garcinia Cambogia*-Extrakt und Carnitin in einem bestimmten Verhältnis eine signifikante Steigerung der sebumreduzierenden Wirkung. Insbesondere eine Kombination des Extraktes mit Carnitin im Verhältnis 1:1 bis etwa 1:2 ist vorteilhaft im Sinne der Erfindung, da sich für diese Verhältnisse synergistische Effekte auf die Sebumreduktion beobachten lassen. Insbesondere zeigte die Kombination Carnitin:*Garcinia Cambogia* Extrakt im Konzentrations-Verhältnis von 1,5:1 bis 1:1 und 1:0,5, und am stärksten im Bereich von 1,5:1 eine synergistische Lipidreduktion.

Vorteilhaft enthalten die erfindungsgemäßen Verwendungen ein Hydroxycitrat:Carnitin-Verhältnis von 1:1 bis 1:4, was etwa einem *Garcinia Cambogia* Extrakt: Carnitin Verhältnis von 1:1 bis 1:2 entspricht.

Die erfindungsgemäßen Verwendungen können in Form von mittels Pinseln oder Abstreifern, Roll-on-Vorrichtungen oder Zerstäubern auftragbaren flüssigen Zusammensetzungen, als Stifte und in Form von aus normalen Flaschen und Behältern auftragbaren Systemen, z. B. Crèmes, Gelen oder Lotionen vorliegen. So können sie z. B. eine Lösung, eine Emulsion vom Typ Wasser-in-Öl (W/O) oder vom Typ Öl-in-Wasser (O/W), oder eine multiple Emulsion, beispielsweise vom Typ Wasser-in-Öl-in-Wasser (W/O/W), Öl-in-Wasser-in-Öl (O/W/O), ein Gel, eine Hydrodispersion, eine lamellare Phase, eine flüssige isotrope Lösungsphase, eine micellare Phase, eine solide oder dispergierte ein- oder mehrfach hexagonale Phase, eine solide oder dispergierte ein- oder mehrfach kubische Phase, eine lyotrope Phase, eine kristalline Phase, einen festen Stift oder auch ein Aerosol darstellen.

Weiterhin können die erfindungsgemäßen Verwendungen vorteilhaft in Form von Gesichtswässern, Tinkturen, Reinigungsformulierungen, Pads, Wattebäuschen oder Tüchern sowie in Form von Tonics oder Shampoos vorliegen.

Vorteilhaft wird der pH-Wert der erfindungsgemäß verwendeten Zubereitungen im schwach sauren bis neutralen Bereich eingestellt, bevorzugt von 3,0 - 7,0, besonders bevorzugt von 5,0 - 6,5.

Die erfindungsgemäß verwendeten Formulierungen können wie üblich zusammengesetzt sein und zur Behandlung der Haut und/oder der Haare im Sinne einer dermatologischen Behandlung oder einer Behandlung im Sinne der pflegenden Kosmetik dienen. Sie können aber auch in Schminkprodukten in der dekorativen Kosmetik eingesetzt werden oder in den kosmetischen und dermatologischen Reinigungsprodukten.

Weiter besonders bevorzugt ist es, wenn zusätzlich ein vernetzter Verdicker in der topischen Zubereitung enthalten ist.

Weiter besonders bevorzugt ist es, wenn der vernetzte Verdicker Ammonium AcryloyldimethyltaurateNP Crosspolymer oder Acrylates/C10-30 Alkyl Acrylate Crosspolymer ist.

Weiter besonders bevorzugt ist es, wenn zusätzlich ein nicht vernetzter Verdicker der topischen Zubereitung enthalten ist.

Weiter besonders bevorzugt ist es, wenn nicht vernetzte Verdicker Xanthan Gum, Carrageenan oder ein Polymer, das überwiegend aus Acrylsäureeinheiten aufgebaut ist, darstellt.

Es ist dem Fachmann natürlich bekannt, dass kosmetische Zubereitungen zumeist nicht ohne die üblichen Hilfs- und Zusatzstoffe denkbar sind. Die erfindungsgemäß verwendeten kosmetischen und dermatologischen Zubereitungen können dementsprechend ferner kosmetische Hilfsstoffe enthalten, wie sie üblicherweise in solchen Zubereitungen verwendet werden; beispielsweise Konsistenzgeber, Konservierungsmittel, Stabilisatoren, Füllstoffe, Parfüme, Pigmente, die färbende Wirkung haben, Verdickungsmittel, Suspendiermittel, Puffergemische, oberflächenaktive Substanzen, Emulgatoren, weichmachende, anfeuchtende und/oder feuchthaltende Substanzen, entzündungshemmende Substanzen, zusätzliche Wirkstoffe wie Vitamine oder Proteine, Lichtschutzmittel, Insektenrepellentien, Bakterizide, Wasser, Salze, antimikrobiell, proteolytische oder keratolytisch wirksame Substanzen, Medikamente oder andere übliche Bestandteile einer kosmetischen oder dermatologischen Formulierung wie Alkohole, Polyole, Polymere, Schaumstabilisatoren, organische Lösungsmittel oder auch Elektrolyte.

Die jeweils einzusetzenden Mengen an Trägerstoffen können in Abhängigkeit von der Art des jeweiligen Produktes vom Fachmann durch einfaches Ausprobieren leicht ermittelt werden.

Als übliche Trägerstoffe zur Herstellung der erfindungsgemäß verwendeten Zubereitungen können neben Wasser, Ethanol und Isopropanol, Glycerin und Propylenglykol hautpflegende Fett- oder fettähnliche Stoffe, wie Ölsäuredecylester, Cetylalkohol, Cetylstearylalkohol und 2-Octyldodecanol, in den für solche Präparate üblichen Mengenverhältnissen eingesetzt werden, sowie schleimbildende Stoffe und Verdickungsmittel, z. B. Hydroxyethyl- oder Hydroxypropylcellulose, Polyacrylsäure, Polyvinylpyrrolidon, daneben aber auch in kleinen Mengen cyclische Silikonöle (Polydimethylsiloxane) sowie flüssige Polymethylphenylsiloxane niedriger Viskosität.

Als Emulgatoren zur Herstellung der erfindungsgemäß verwendeten Zubereitungen, welche vorteilhaft als flüssige oder feste Zubereitungen auf die gewünschten Hautbereiche aufgetragen werden sollen, und die in den Zubereitungen in geringer Menge, z.B. 1 bis 6 Gew.-%, bezogen auf die Gesamt-Zusammensetzung, verwendet werden können, haben sich nichtionogene Typen, wie Polyoxyethylen-Fettalkoholether, z. B. Cetostearylalkoholpolyethylenglykolether mit 12 bzw. 20 angelagerten Ethylenoxid-Einheiten pro Molekül, Cetostearylalkohol sowie Sorbitanester und Sorbitanester-Ethylenoxid-Verbindungen (z. B. Sorbitanmonostearat und Polyoxyethylensorbitanmonostearat) und langkettige höhermolekulare wachsartige Polyglykolether als geeignet erwiesen. Daneben sind aber auch eine ganze Reihe von anderen Emulgatoren oder Emulgatormischungen geeignet, welche üblichweise in kosmetischen Zubereitungen Verwendung finden. Dazu zählen beispielsweise, aber nicht beschränkend auf, Glycerylstearatcitrat, PEG-40 Stearat oder auch Polyglyceryl(3)-Methylglucosedistearat, Stearinsäure, Steareth-2 und Steareth-21.

Die Ölphase der erfindungsgemäß verwendeten Zubereitungen wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen.

Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Verbindungen der Ölphase können synthetischer, halbsynthetischer oder natürlicher Herkunft sein.

Auch beliebige Abmischungen solcher Öl- und Wachskomponenten sind vorteilhaft im Sinne der vorliegenden Erfindung einzusetzen.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft beträgt der Gehalt an der Ölphase zwischen 1 und 50 Gew.-%, bezogen auf das Gesamtgewicht der Zubereitungen, bevorzugt 2 - 30 Gew.-%, insbesondere bevorzugt unter 10 Gew.-%.

Es ist bevorzugt, den beschriebenen Emulsionen Komplexbildner zuzufügen. Komplexbildner sind an sich bekannte Hilfsstoffe der Kosmetologie bzw. der medizinschen Galenik. Komplexbildner, insbesondere Chelatoren bilden mit Metallatomen Komplexe, welche bei Vorliegen eines oder mehrerer mehrbasiger Komplexbildner, also Chelatoren, Metallacyclen darstellen. Chelate stellen Verbindungen dar, in denen ein einzelner Ligand mehr als eine Koordinationsstelle an einem Zentralatom besetzt. In diesem Falle werden also normalerweise gestreckte Verbindungen durch Komplexbildungen über ein Metall-Atom oder -Ion zu Ringen geschlossen. Die Zahl der gebundenen Liganden hängt von der Koordinatinszahl des zentralen Metalls ab. Voraussetzung für die Chelatbildung ist, dass die mit dem Metall reagierende Verbindung zwei oder mehrere Atomgruppierungen enthält, die als Elektronendonatoren wirken.

Der oder die Komplexbildner können vorteilhaft aus der Gruppe der üblichen Verbindungen gewählt werden, wobei bevorzugt mindestens eine Substanz aus der Gruppe bestehend aus Weinsäure und deren Anionen, Citronensäure und deren Anionen, Aminopolycarbonsäuren und deren Anionen (wie beispielsweise Ethylendiamintetraessigsäure (EDTA) und deren Anionen, sowie Nitrilotriessigsäure (NTA) und deren Anionen).

Im Sinne der vorliegenden Erfindung sind insbesondere chelatisierende Komplexbildner bevorzugt, da sie durch Komplexierung und Chelatisierung der zweiwertigen Metallionen wie beispielsweise Ca²⁺, die Penetration von Hydroxycitrat in die Haut verbessern können.

Ein bevorzugter Komplexbildner im Sinne der Erfindung ist Ethylendiamintetraessigsäure (EDTA), die mit der INCl-Bezeichnung Trisodium EDTA eingesetzt wird.

Der oder die Komplexbildner sind vorteilhaft in kosmetischen oder dermatologischen Zubereitungen bevorzugt zu 0,01 Gew.-% bis 10 Gew.-%, bevorzugt zu 0,05 Gew.-% bis 10 Gew.-%, insbesondere bevorzugt zu 0,1-1,0 Gew.-%, bezogen auf das Gesamtgewicht der Zubereitungen, enthalten.

Vorteilhafte Konservierungsmittel im Sinne der vorliegenden Erfindung sind beispielsweise Benzylalkohol, Sorbinsäure und dessen Salze, Formaldehydabspalter (wie z. B. Diazolidinylharnstoff (Handelsbezeichnung Germall II von ISP), Imidazolidinylharnstoff (Handelsbezeichnung Germall 115) oder DMDM Hydantoin, welches beispielsweise unter der Handelsbezeichnung Glydant™ von der Fa. Lonza erhältlich ist), Methylisothiazolinon und entsprechende Derivate (Handelsname Kathon CG), lodopropylbutylcarbamate (z. B. die unter den Handelsbezeichnungen Glycacil-L, Glycacil-S von der Fa. Lonza erhältlichen und/oder Dekaben LMB von Jan Dekker), Parabene (d. h. p-Hydroxybenzoesäurealkylester, wie Methyl-, Ethyl-, Propyl-, Isopropyl-, und/oder Butylparaben), Phenoxyethanol, Ethanol, Triclosan, Benzoesäure und dergleichen mehr. Üblicherweise umfaßt das Konservierungssystem ferner vorteilhaft auch Substanzen, die die Wirksamkeit von klassischen Konservierungsmitteln verbessern, wie beispielsweise Hexandiol, Pentandiol, Butylenglycol oder auch Methylpropandiol.

Es ist ebenfalls vorteilhaft, den Zubereitungen im Sinne der vorliegenden Erfindung übliche Antioxidantien zuzufügen. Es können als günstige Antioxidantien alle für kosmetische und/oder dermatologische Anwendungen geeigneten oder gebräuchlichen Antioxidantien verwendet werden, insbesondere bevorzugt sind alpha-Glycosylrutin (AGR) und/oder Taxifolin.

Die Menge der Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Gelbildner, auch Verdickungsmittel genannt, sind Makromoleküle, die eine weitgehend lineare Gestalt haben und über intermolekulare Wechselwirkungskräfte verfügen, die Neben- und Hauptvalenzbindungen zwischen den einzelnen Molekülen und damit die Ausbildung eines netzartigen Gebildes ermöglichen. Sie sind teilweise wasserlösliche natürliche oder synthetische Polymere, die in wässrigen Systemen Gele oder viskose Lösungen bilden. Sie erhöhen die Viskosität des Wassers, indem sie entweder Wassermoleküle binden (Hydratation) oder aber das Wasser in ihre unter sich verflochtenen Makromoleküle aufnehmen und einhüllen, wobei sie gleichzeitig die Beweglichkeit des Wassers einschränken. Solche wasserlöslichen Polymere stellen eine große Gruppe chemisch sehr unterschiedlicher natürlicher und synthetischer Polymere dar, deren gemeinsames Merkmal ihre Löslichkeit in Wasser bzw. wässrigen Medien ist. Voraussetzung dafür ist, dass diese Polymere über eine für die Wasserlöslichkeit ausreichende Anzahl an hydrophilen Gruppen besitzen und nicht zu stark vernetzt sind. Die hydrophilen Gruppen können nichtionischer, anionischer oder kationischer Natur sein.

Durch Experimente konnte gezeigt werden, dass für die stabile Formulierung von Hydroxycitrat alleine und besonders in Kombination mit Carnitin insbesondere die Verwendung von quervernetzenden Gelbildnern als Verdickungsmittel besonders geeignet bzw. sogar notwendig sind. Darüberhinaus haben sich Kombinationen von bestimmten Gelbildnern als besonders geeignet herausgestellt, mit denen die genannten Wirkstoffe bzw. Wirkstoffkombinationen in kosmetische und dermatologische Zubereitungen stabil eingearbeitet werden können. Die üblicherweise in kosmetischen Zubereitungen verwendeten Carbomere sind alleine nicht in der Lage, stabile und kosmetisch ansprechende Zubereitungen mit denen in dieser Erfindung aufgeführten Wirkstoffen zu bilden.

Zu den geeigneten quervernetzenden Gelbildnern, mit denen stabile und sensorisch ansprechende kosmetische Zubereitungen hergestellt werden konnten, gehören die Copolymere aus C₁₀₋₃₀-Alkylacrylaten und einem oder mehreren Monomeren der Acrylsäure, der Methacrylsäure oder deren Ester, die kreuzvernetzt sind mit einem Allylether der Saccharose oder einem Allylether des Pentaerythrit.

Vorteilhaft sind Verbindungen, die die INCI-Bezeichnung "Acrylates/C₁₀₋₃₀ Alkyl Acrylate Crosspolymer" tragen. Insbesondere vorteilhaft sind die unter den Handelsbezeichnungen Pemulen® TR1 und Pemulen® TR2 bei der Firma Noveon erhältlichen.

Desweiteren sind besonders vorteilhaft Verbindungen, die die INCI-Bezeichnung Ammoniumacryloyldimethyltaurate/Vinylpyrrolidoncopolymere tragen.

Erfindungsgemäß vorteilhaft weisen das oder die Ammoniumacryloyldimethyltaurate/Vinylpyrrolidoncopolymere die Summenformel [C₇H₁₆N₂SO₄]ₙ [C₆H₉NO]ₘ auf, einer statistischen Struktur wie folgt entsprechend

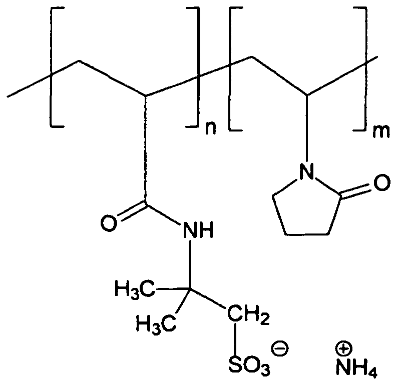

Bevorzugte Spezies im Sinne der vorliegenden Erfindung sind in den Chemical Abstracts unter den Registraturnummern 58374-69-9, 13162-05-5 und 88-12-0 abgelegt und erhältlich unter der Handelsbezeichnung Aristoflex® AVC der Gesellschaft Clariant GmbH. Vorteilhaft sind ferner Copolymere/Crosspolymere umfassend Acryloyldimethyl Taurate, wie beispielsweise Simugel® EG oder Simugel® EG von der Gesellschaft Seppic S.A.

Als sogenannter Co-Gelbildner im Sinne der vorliegenden Erfindung bevorzugt ist Xanthan (CAS-Nr. 11138-66-2), auch Xanthan Gummi genannt, welches ein anionisches Heteropolysaccharid ist, das in der Regel durch Fermentation aus Maiszucker gebildet und als Kaliumsalz isoliert wird. Es wird von Xanthomonas campestris und einigen anderen Species unter aeroben Bedingungen mit einem Molekulargewicht von 2×10⁶ bis 24×10⁶ produziert. Xanthan wird aus einer Kette mit β-1,4-gebundener Glucose (Cellulose) mit Seitenketten gebildet. Die Struktur der Untergruppen besteht aus Glucose, Mannose, Glucuronsäure, Acetat und Pyruvat. Die Anzahl der Pyruvat-Einheiten bestimmt die Viskosität des Xanthans.

Ein weiterer vorteilhafter Co-Gelbildner im Sinne der vorliegenden Erfindung ist ferner Carrageen, ein gelbildender und ähnlich wie Agar aufgebauter Extrakt aus nordatlantischen, zu den Florideen zählenden Rotalgen (Chondrus crispus und Gigartina stellata).

Häufig wird die Bezeichnung Carrageen für das getrocknete Algenprodukt und Carrageenan für den Extrakt aus diesem verwendet. Das aus dem Heißwasserextrakt der Algen ausgefällte Carrageen ist ein farbloses bis sandfarbenes Pulver mit einem Molekulargewichtsbereich von 100 000-800 000 und einem Sulfat-Gehalt von ca. 25 %. Carrageen ist in warmem Wasser sehr leicht löslich und bildet beim Abkühlen ein thixotropes Gel, selbst wenn der Wassergehalt 95-98 % beträgt. Die Festigkeit des Gels wird durch die Doppelhelix-Struktur des Carrageens bewirkt. Beim Carrageenan unterscheidet man drei Hauptbestandteile: Die gelbildende κ-Fraktion besteht aus D-Galactose-4-sulfat und 3,6-Anhydro-α-D-galactose, die abwechselnd in 1,3- und 1,4-Stellung glykosidisch verbunden sind (Agar enthält demgegenüber 3,6-Anhydro-α-L-galactose). Die nicht gelierende λ-Fraktion ist aus 1,3-glykosidisch verknüpften D-Galactose-2-sulfat und 1,4-verbundenen D-Galactose-2,6-disulfat-Resten zusammengesetzt u. in kaltem Wasser leicht löslich. Das aus D-Galactose-4-sulfat in 1,3-Bindung und 3,6-Anhydro-α-D-galactose-2-sulfat in 1,4-Bindung aufgebaute 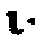 -Carrageenan ist sowohl wasserlöslich als auch gelbildend. Weitere Carrageen-Typen werden ebenfalls mit griechischen Buchstaben bezeichnet: alpha, beta, gamma, my, ny, zeta, pi, omega, chi. Auch die Art vorhandener Kationen (K⁺, NH₄⁺, Na⁺, Mg²⁺, Ca²⁺) beeinflusst die Löslichkeit der Carrageene.

Darüber hinaus sind eine ganze Reihe weiterer Hydrokolloide bekannt und ebenfalls als Co-Gelbildner geeignet.

Es ist auch möglich und vorteilhaft im Sinne der vorliegenden Erfindung, einen Gehalt an UV-Schutzsubstanzen zu verwenden. Vorteilhaft können daher erfindungsgemäße Zubereitungen Substanzen enthalten, die UV-Strahlung im UVB-Bereich absorbieren, wobei die Gesamtmenge der Filtersubstanzen vozugsweise zwischen 0,1 Gew.-% bis 30 Gew.-% betragen kann. Bevorzugt sind handelsübliche wasser- oder öllösliche UVB-Filter. Es kann auch von Vorteil sein, in erfindungsgemäß verwendeten Zubereitungen UVA-Filter einzusetzen, die üblicherweise in kosmetischen und/oder dermatologischen Zubereitungen enthalten sind. Es können die gleichen Mengen eingesetzt werden, welche für UVB-Filter genannt wurden.

Kosmetische und/oder dermatologische Zubereitungen, die im Sinne der vorliegenden Erfindung verwendet werden, können auch anorganische Pigmente enthalten, die üblicherweise in der Kosmetik zum Schutz der Haut vor UV-Strahlen verwendet werden. Dabei handelt es sich üblicherweise um Oxide des Titans, Zinks, Eisens, Zirkoniums, Siliciums, Mangans, Aluminiums und Mischungen davon. Besonders bevorzugt handelt es sich um Pigmente auf der Basis von Titandioxid. Es können die für die vorherstehenden Kombinationen genannten Mengen verwendet werden.

Weiterhin bevorzugt im Sinne der vorliegenden Erfindung ist es, den kosmetischen und/oder dermatologischen Zubereitungen Füllstoffe oder Puderrohstoffe zuzufügen. Die Stoffe verbessern die sensorischen Eigenschaften der Zubereitungen und haben darüber hinaus mattierende Eigenschaften, wie sie insbesondere bei Zubereitungen gegen fettige und/oder unreine Haut erwünscht sind. Zu den üblicherweise verwendeten Stoffen gehören beispielsweise Tapioca Starch, Distarch Phosphate, Aluminium Starch Octenylsuccinate, Silica, Mikrokristalline Cellulose, Talc, Kaolin, Cyclodextrine, Bentonite, Titandioxid und andere.

Die erfindungsgemäß verwendeten Wirkstoffe können auch besonders vorteilhaft in Mikroemulsionen und/oder Nanoemulsionen, beispielsweise wie in der deutschen Offenlegungsschrift DE-195 9 079 beschrieben, verwendet werden.

Zur Anwendung werden die erfindungsgemäß verwendeten kosmetischen und/oder dermatologischen Formulierungen in der für Kosmetika und Dermatika üblichen Weise auf die Haut, Kopfhaut und/oder die Haare in ausreichender Menge aufgebracht.

Die folgenden Beispiele sollen die Verkörperungen der vorliegenden Erfindungen verdeutlichen.

**Rezepturbeispiel 1: (Zubereitung gegen fettige/unreine Haut)**

| Rohstoff (INCI) | Gew.-% |
|---|---|
| Glyceryl Stearate Citrate | 1,50 |
| Caprylic/Capric Triglyceride | 1,00 |
| Octyldodecanol | 1,00 |
| Myristyl Myristate | 1,00 |
| Dimethicone | 0,50 |
| Cetearyl Alcohol | 0,50 |
| Glycerin | 7,50 |
| Xanthan Gum | 0,20 |
| Ammonium AcryldimethyltaurateNP Copolymer | 0,50 |
| Tocopheryl Acetate | 0,50 |
| Garcinia Cambogia Extract (Hydroxycitrat) | 1,00 |
| Carnitin | 1,00 |
| Alcohol, Denat. | 3,00 |
| Parfum, Konservierungsmittel | q.s. |
| Aqua | Ad.100 |

**Rezepturbeispiel 2: (Zubereitung gegen fettige/unreine Haut)**

| Rohstoff (INCI) | Gew.-% |
|---|---|
| Glyceryl Stearate Citrate | 1,50 |
| Caprylic/Capric Triglyceride | 1,00 |
| Octyldodecanol | 1,00 |
| Myristyl Myristate | 1,00 |
| Dimethicone | 0,50 |
| Cetearyl Alcohol | 0,50 |
| Glycerin | 7,50 |
| Xanthan Gum | 0,20 |
| Ammonium AcryldimethyltaurateNP Copolymer | 0,50 |
| Tocopheryl Acetate | 0,50 |
| Distarch Phosphate | 4,00 |
| Trisodium EDTA | 1,00 |
| Aluminum Chlorohydrate | 0,02 |
| Garcinia Cambogia Extract (Hydroxycitrat) | 1,00 |
| Carnitin | 2,00 |
| Alcohol, Denat. | 3,00 |
| Parfum, Konservierungsmittel | q.s. |
| Aqua | Ad. 100 |

**Rezepturbeispiel 3: (Zubereitung gegen fettige/unreine Haut)**

| Rohstoff (INCI) | Gew.-% |
|---|---|
| PEG-40 Stearate | 1,30 |
| Glyceryl Stearate | 2,70 |
| Paraffinum Liquidum | 6,00 |
| Caprylic/Capric Triglyceride | 3,00 |
| Cetearyl Isononanoate | 3,00 |
| Cetyl Alcohol | 4,00 |
| Dimethicone | 3,00 |
| Glycerin | 10,00 |
| Carbomer | 0,20 |
| Garcinia Cambogia Extract (Hydroxycitrat) | 1,00 |
| Carnitin | 1,00 |
| Alcohol, Denat. | 3,00 |
| Parfum, Konservierungsmittel | q.s. |
| Aqua | Ad. 100 |

**Rezepturbeispiel 4: (Zubereitung gegen fettige/unreine Haut)**

| Rohstoff (INCI) | Gew.-% |
|---|---|
| Polyglyceryl-3 Methylglucose Distearate | 2,25 |
| Sorbitan Stearate | 0,75 |
| C12-15 Alkyl Benzoate | 2,50 |
| Stearyl Alcohol | 0,50 |
| Cyclomethicone | 2,00 |
| Glycerin | 4,32 |
| Octocrylene | 1,50 |
| Carbomer | 0,20 |
| Ammonium Acryldimethyltaurate/VP Copolymer | 0,50 |
| Garcinia Cambogia Extract (Hydroxycitrat) | 1,00 |
| Carnitin | 1,00 |
| Titanium Dioxide + Alumina + Silica + Sodium Polyacrylate | 1,20 |
| Trisodium EDTA | 1,00 |
| Aluminum Chlorohydrate | 0,04 |
| Alcohol, Denat. | 2,00 |
| Parfum, Konservierungsmittel | q.s. |
| Aqua | Ad. 100 |

**Rezepturbeispiel 5: (Zubereitung gegen fettige/unreine Haut, nicht erfindungsgemäß)**

| Rohstoff (INCI) | Gew.-% |
|---|---|
| Polyglyceryl-3 Methylglucose Distearate | 2,25 |
| Sorbitan Stearate | 0,75 |
| C12-15 Alkyl Benzoate | 2,50 |
| Stearyl Alcohol | 0,50 |
| Cyclomethicone | 2,00 |
| Glycerin | 4,32 |
| Octocrylene | 1,50 |
| Carbomer | 0,20 |
| Ammonium Acryldimethyltaurate/VP Copolymer | 0,50 |
| Garcinia Cambogia Extract (Hydroxycitrat) | 1,00 |
| Titanium Dioxide + Alumina + Silica + Sodium Polyacrylate | 1,20 |
| Trisodium EDTA | 1,00 |
| Aluminum Chlorohydrate | 0,04 |
| Alcohol, Denat. | 2,00 |
| Parfum, Konservierungsmittel | q.s. |
| Aqua | Ad. 100 |

**Rezepturbeispiel 6: (Zubereitung gegen fettige/unreine Haut)**

| Rohstoff (INCI) | Gew.-% |
|---|---|
| Polyglyceryl-3 Methylglucose Distearate | 2,25 |
| Sorbitan Stearate | 0,75 |
| C12-15 Alkyl Benzoate | 2,50 |
| Stearyl Alcohol | 0,50 |
| Cyclomethicone | 2,00 |
| Glycerin | 4,32 |
| Acrylates/C10-30 Alkyl Acrylate Crosspolymer | 0,20 |
| Ammonium AcryldimethyltaurateNP Copolymer | 0,50 |
| Distarch Phosphate | |
| Garcinia Cambogia Extract (Hydroxycitrat) | 1,00 |
| Carnitin | 2,00 |
| Alcohol, Denat. | 2,00 |
| Parfum, Konservierungsmittel | q.s. |
| Aqua | Ad. 100 |

**Rezepturbeispiel 7: (Zubereitung gegen fettige/unreine Haut)**

| Rohstoff (INCI) | Gew.-% |
|---|---|
| PEG-40 Stearate | 2,50 |
| Glyceryl Stearate | 2,25 |
| Caprylic/Capric Triglyceride | 4,00 |
| C12-15 Alkyl Benzoate | 0,50 |
| Octyldodecanol | 0,10 |
| PEG-8 | 1,00 |
| Cetyl Alcohol | 2,50 |
| Glycerin | 5,00 |
| Octocrylene | 1,50 |
| Cyclomethicone | 5,00 |
| Tocopheryl Acetate | 0,50 |
| Garcinia Cambogia Extract (Hydroxycitrat) | 1,00 |
| Carnitin | 1,00 |
| Trisodium EDTA | 1,00 |
| Talc | 0,50 |
| Distarch Phosphate | 4,00 |
| Alcohol, Denat. | 2,00 |
| Parfum, Konservierungsmittel | q.s. |
| Aqua | Ad. 100 |

**Rezepturbeispiel 8: (Zubereitung gegen fettige/unreine Haut)**

| Rohstoff (INCI) | Gew.-% |
|---|---|
| PEG-40 Stearate | 1,00 |
| Glyceryl Stearate | 2,50 |
| C12-15 Alkyl Benzoate | 1,00 |
| Octyldodecanol | 0,50 |
| Cetearyl Alcohol | 2,50 |
| Simmondsia Chinensis | 0,50 |
| Glycerin | 5,00 |
| Ethylhexyl Methoxycinnamate + BHT | 7,50 |
| Tapioca Starch + Aqua | 4,00 |
| Carbomer | 0,10 |
| Ammonium Acryldimethyltaurate/VP Copolymer | 0,50 |
| Garcinia Cambogia Extract (Hydroxycitrat) | 1,00 |
| Carnitin | 2,00 |
| Parfum, Konservierungsmittel | q.s. |
| Aqua | Ad. 100 |

**Rezepturbeispiel 9: (Zubereitung gegen fettige/unreine Haut)**

| Rohstoff (INCI) | Gew.-% |
|---|---|
| PEG-40 Stearate | 1,00 |
| Glyceryl Stearate | 2,50 |
| C12-15 Alkyl Benzoate | 1,00 |
| Octyldodecanol | 0,50 |
| Cetearyl Alcohol | 2,50 |
| Simmondsia Chinensis | 0,50 |
| Glycerin | 5,00 |
| Ethylhexyl Methoxycinnamate + BHT | 7,50 |
| Tapioca Starch + Aqua | 4,00 |
| Benzophenone-3 | 3,00 |
| Simmondsia Chinensis | 0,50 |
| Carbomer | 0,15 |
| Ammonium AcryldimethyltaurateNP Copolymer | 0,50 |
| Garcinia Cambogia Extract (Hydroxycitrat) | 1,00 |
| Carnitin | 2,00 |
| Parfum, Konservierungsmittel | q.s. |
| Aqua | Ad. 100 |

**Rezepturbeispiel 10: (Zubereitung für Haarpflegeprodukte: Haarwasser)**

| | **1(*)** | **2** | **3** | **4** |
|---|---|---|---|---|
| Ethanol | 30,0 | 50,0 | - | - |
| Isopropanol | - | - | 40,0 | 30,0 |
| Panthenol | 0,2 | 0,1 | 0,2 | 0,2 |
| Menthol | 0,1 | - | 0,05 | 0,05 |
| Tocopheryl Acetate | 0,2 | 0,2 | - | 0,1 |
| C12-13 Alkyl Lactate | 0,2 | 0,1 | 0,2 | - |
| Garcinia Cambogia Extract (Hydroxycitrat) | 1,00 | 1,00 | 1,00 | 1,00 |
| Carnitin | - | 1,00 | 1,00 | 1,00 |
| Parfüm, Konservierungsmittel | q.s. | q.s. | q.s. | q.s. |
| Wasser | ad 100 | ad 100 | ad 100 | ad 100 |

| | | | | |
|---|---|---|---|---|
| (*) nicht erfindungsgemäß | | | | |

**Rezepturbeispiel 11: (Zubereitung für Haarpflegeprodukte: Haarstyling Gel)**

| | **1** | **2** | **3** |
|---|---|---|---|
| PVPNA Copolymer | 5,0 | 6,0 | 7,0 |
| Carbomer | 0,5 | | 0,8 |
| Acrylatcopolymer (Acrylates/C 10-30 Alkyl Acrylate Crosspolymer) | - | 1,0 | - |
| PEG-40 hydriertes Rizinusöl | 0,2 | 0,2 | 0,2 |
| Silikonöl | - | - | 0,1 |
| Glycerin | 3,0 | - | - |
| NaOH | q.s. | q.s. | q.s. |
| Parfum | 0,3 | 0,3 | 0,3 |
| Garcinia Cambogia Extract (Hydroxycitrat) | 1,00 | 1,00 | 1,00 |
| Carnitin | 2,00 | 2,00 | 2,00 |
| Ethanol | - | - | 10,0 |
| Wasser | ad 100 | ad 100 | ad 100 |

**Rezepturbeispiel 12: (Zubereitung für Haarpflegeprodukte: Schaumfestiger)**

| | Schaumfestiger starke Festigung | Schaumfestiger extra starke Festigung |
|---|---|---|
| | **16** | **17** |
| Acrylates Copolymer (4) | 2,00 | 2,00 |
| Cocamidopropylbetain | 0,50 | 0,50 |
| Parfüm, Konservierungsmittel, pH-Einstellung, Lösungsvermittler | q.s. | q.s. |
| Propan/Butan | 8,00 | 8,00 |
| Garcinia Cambogia Extract (Hydroxycitrat) | 1,00 | 1,00 |
| Carnitin | 2,00 | 2,00 |
| Wasser, VES | ad 100,00 | |

| | | |
|---|---|---|
| (4) anionisches Polymer z.B. von BASF: Luvimer MAEX | | |

**Rezepturbeispiel 13: (Zubereitung für Haarpflegeprodukte: Stylinggel)**

| | Stylinggel starke Festigung | Stylinggel extra starke Festigung |
|---|---|---|
| | **18** | **19** |
| PVPNA Copolymer | 2,00 | 4,00 |
| Acrylates Copolymer (4) | 2,00 | 2,00 |
| Carbomer | 0,50 | 0,50 |
| Parfüm, Konservierungsmittel, pH-Einstellung, Lösungsvermittler | q.s. | q.s. |
| Propylenglycol | 5,00 | 5,00 |
| Garcinia Cambogia Extract (Hydroxycitrat) | 1,00 | 1,00 |
| Carnitin | 1,00 | 1,00 |
| Wasser, VES | ad 100,00 | |

| | | |
|---|---|---|
| (4) anionisches Polymer z.B. von BASF: Luvimer MAEX pH einstellen auf 6,0 | | |

**Rezepturbeispiel 14: (Zubereitung für Haarpflegeprodukte: Haarkur)**

| | **1** | **2** | **3** | **4** | **5 (*)** | **6 (*)** |
|---|---|---|---|---|---|---|
| Hydroxypropylmethylcellulose | 0,5% | 0,5% | 0,5% | 0,3% | 0,4% | 0,5% |
| Cetrimoniumbromid | 1,0 | - | 0,8 | - | 0,5 | 0,7 |
| Behentrimoniumchlorid | - | 0,7 | 0,3 | - | - | - |
| Distearoylethyl Hydroxyethylmonium Methosulfate | Metho- - | - | - | 1,2 | - | 0,7 |
| Palmitamidopropyltrimonium Chlorid | - | - | - | - | 0,5 | - |
| Glycerin | 3,0 | 3,0 | 3,0 | 2,5 | 2,0 | - |
| Cetearylalkohol | 2,5 | 2,5 | 2,5 | 2,5 | 2,0 | 2,5 |
| Glycerylstearat | 2,0 | 2,0 | 2,0 | 2,0 | 2,0 | 1,8 |
| Polyquaternium-10 | 0,1 | - | - | - | 0,1 | - |
| Guar Hydroxypropyl Trimonium Chlorid | - | 0,2 | - | 0,1 | - | - |
| Garcinia Cambogia Extract (Hydroxycitrat) | 1,00 | 1,00 | 1,00 | 1,00 | 1,00 | 1,00 |
| Carnitin | 2,00 | 2,00 | 2,00 | 2,00 | - | 2,00 |
| Konservierungsmittel, Parfüm, Lösungsvermittler | q.s. | q.s. | q.s. | q.s. | q.s. | q.s. |
| Wasser | ad 100 | ad 100 | ad 100 | ad 100 | ad 100 | ad 100 |

| | | | | | | |
|---|---|---|---|---|---|---|
| (*) nicht erfindungsgemäß | | | | | | |

**Rezepturbeispiel 15: (Zubereitung für Haarpflegeprodukte: Haarspülung)**

| | **1** | **2** | **3** | **4** | **5** | **6** |
|---|---|---|---|---|---|---|
| Cetrimoniumchlorid | 1,0 | 0,5 | 0,5 | - | 0,5 | 0,5 |
| Distearoylethyl Hydroxyethylmonium Methosulfate | - | - | - | 1,0 | - | 0,5 |
| Palmitamidopropyltrimonium Chlorid | - | - | - | - | 0,8 | - |
| Behentrimoniumchlorid | - | 0,2% | 0,3 | - | - | - |
| Cetearylalkohol | 3,0 | 2,5 | 2,8 | 3,0 | 2,6 | 2,8 |
| Glycerin | 3,0 | 3,0 | 3,0 | 2,8 | 2,5 | 2,0 |
| Hydroxyethylcellulose | 0,2 | 0,2 | 0,2 | 0,2 | 0,2 | 0,2 |
| Polyquaternium-10 | 0,1 | - | - | - | - | 0,1 |
| Guar Hydroxypropyl Trimonium Chlorid | - | 0,2 | - | - | - | - |
| Garcinia Cambogia Extract (Hydroxycitrat) | 1,00 | 1,00 | 1,00 | 1,00 | 1,00 | 1,00 |
| Carnitin | 1,00 | 1,00 | 1,00 | 1,00 | 1,00 | 1,00 |
| Konservierungsmittel, Parfüm, Lösungsvermittler | q.s. | q.s. | q.s. | q.s. | q.s. | q.s. |
| Wasser | ad 100 | ad 100 | ad 100 | ad 100 | ad 100 | ad 100 |

**Rezepturbeispiel 16: (Zubereitung für Haarpflegeprodukte: Spray-Conditioner)**

| | **1** | **2** | **3** | **4** | **5** | **6** | **7** |
|---|---|---|---|---|---|---|---|
| Cetrimoniumchlorid | 0,2 | 0,1 | 0,8 | 0,5 | - | 0,2 | - |
| Behentrimoniumchlorid | - | 0,2 | 0,3 | - | - | - | - |
| Distearoylethyl Hydroxyethylmonium Methosulfate | - | - | - | - | 0,2 | - | 0,2 |
| Palmitamidopropyltrimonium Chlorid | - | - | - | - | - | 0,1 | 0,1 |
| Benzophenone-4 | 0,05 | 0,03 | - | - | - | - | 0,03 |
| PVP/VA Copolymer | - | 0,7 | - | - | - | 0,2 | - |
| Polyquaternium-10 | 0,1 | - | - | - | 0,1 | - | - |
| Polyquaternium-4 | 0,2 | - | - | - | - | 0,2 | 0,2 |
| Propylene Glycol | - | - | - | 3,0 | 2,0 | - | 2,0 |
| Polyquaternium-11 | - | - | 0,2 | - | - | - | - |
| Panthenol | 0,1 | - | 0,2 | 0,1 | - | 0,2 | - |
| Glyceryl Isostearate | - | - | - | 0,4 | - | - | - |
| Isoceteth-20 | - | - | - | 0,8 | - | - | - |
| Dicaprylyl Carbonate | - | - | - | 0,5 | - | - | - |
| Garcinia Cambogia Extract (Hydroxycitrat) | 1,00 | 1,00 | 1,00 | 1,00 | 1,00 | 1,00 | 1,00 |
| Carnitin | 2,00 | 2,00 | 2,00 | 2,00 | 2,00 | 2,00 | 2,00 |
| Konservierungsmittel, Parfüm, Lösungsvermittler | q.s. | q.s. | q.s. | q.s. | q.s. | q.s. | q.s. |
| Wasser | ad 100 | ad 100 | ad 100 | ad 100 | ad 100 | ad 100 | ad 100 |

**Rezepturbeispiel 17: (Zubereitung für Haarpflegeprodukte: Leave-on Conditioner)**

| | **1** | **2** | **3** | **4** | **5** | **6** | **7** |
|---|---|---|---|---|---|---|---|
| Cetylalcohol | 1,5 | 1,8 | 2,0 | - | 1,0 | 1,7 | 2,0 |
| Cetrimoniumchlorid | 0,3 | 0,1 | 0,5 | 0,5 | - | 0,2 | - |
| Behentrimoniumchlorid | - | 0,2 | - | - | - | - | 0,2 |
| Distearoylethyl Hydroxyethylmonium Methosulfate | - | - | - | - | 0,5 | - | 0,2 |
| Palmitamidopropyltrimonium Chlorid | - | - | - | - | - | 0,2 | - |
| Benzophenone-4 | 0,05 | 0,03 | - | 0,1 | - | 0,1 | - |
| PVPNA Copolymer | 0,4 | - | - | - | 0,3 | - | - |
| Polyquaternium-37 | - | - | - | 1,0 | - | - | - |
| Polyquaternium-4 | - | - | - | 0,2 | - | 0,1 | - |
| Polyquaternium-10 | - | - | 0,5 | - | 0,3 | - | 0,2 |
| Panthenol | 0,1 | - | 0,2 | 0,1 | - | - | 0,2 |
| Hydroxyethylcellulose | - | - | - | 0,3 | - | - | 0,2 |
| Acrylates/C10-30 Alkyl Acrylates Crosspolymer | 0,5 | 0,3 | 0,2 | - | - | - | - |
| C12-13 Alkyl Lactate | 2,0 | 1,0 | 1,5 | 1,0 | 1,5 | 1,0 | 1,0 |
| Laureth-4 | - | - | - | 0,5 | - | - | - |
| Aluminium Starch Octenylsuccinate | - | - | - | 1,0 | - | - | - |
| Dicaprylyl Carbonate | - | - | - | 1,0 | - | - | - |
| Garcinia Cambogia Extract (Hydroxycitrat) | 1,00 | 1,00 | 1,00 | 1,00 | 1,00 | 1,00 | 1,00 |
| Carnitin | 1,00 | 1,00 | 1,00 | 1,00 | 1,00 | 1,00 | 1,00 |
| Konservierungsmittel, Parfüm, Lösungsvermittler | q.s. | q.s. | q.s. | q.s. | q.s | q.s | q.s |
| Wasser | ad 100 | ad 100 | ad 100 | ad 100 | ad 100 | ad 100 | ad 100 |

**Rezepturbeispiel 18: (Zubereitung für Haarpflegeprodukte: Conditioner-Shampoo mit Perlglanz)**

| | **1** | **2** | **3 (*)** | **4** | **5** | **6** |
|---|---|---|---|---|---|---|
| Natrium Laurylethersulfat | 9 | 9 | 9 | 9 | 9 | 9 |
| Cocamidopropyl Betain | 4 | 3 | 4 | 3 | 4 | 3 |
| Dinatrium PEG-5 Laurylcitrat Sulfosuccinat | 3 | 3 | 3 | 2 | 3 | 4 |
| Verdicker | 0.1 | 0.1 | 0.1 | 0.2 | 0,3 | 0,4 |
| Polyquaternium-10 | 0.3 | 0.1 | 0.1 | 0.3 | - | 0,2 |
| Guar Hydroxypropyl-Trimonium Chlorid | - | - | 0.1 | 0.1 | 0.2 | 0,3 |
| Perlglanz | 1.5 | 3 | 4 | 2 | 2,5 | 0,75 |
| Trübungsmittel | - | - | - | - | 0.5 | 0,5 |
| Iminodibersteinsäure | 0.1 | 0.2 | 0.1 | 0.5 | 0.5 | 0,5 |
| PEG-40 hydriertes Rizinusöl | 0.2 | 0.3 | 0.4 | 0.5 | 0.2 | 0,3 |
| Natrium Salicylat | 0.2 | 0.2 | 0.4 | 0.4 | 0.4 | 0,2 |
| Natrium Benzoat | 0.4 | 0.4 | 0.4 | 0.4 | 0.4 | 0,4 |
| Natriumchlorid | 0,9 | 1,0 | 1,2 | 1,5 | - | 1,5 |
| Garcinia Cambogia Extract (Hydroxycitrat) | 1,00 | 1,00 | 1,00 | 1,00 | 1,00 | 1,00 |
| Carnitin | 2,00 | 2,00 | - | 2,00 | 2,00 | 2,00 |
| Zitronensäure | q.s. | q.s. | q.s. | q.s. | q.s. | q.s. |
| Parfüm | q.s. | q.s. | q.s. | q.s. | q.s. | q.s. |
| Wasser | ad 100 | ad 100 | ad 100 | ad 100 | ad 100 | ad 100 |

| | | | | | | |
|---|---|---|---|---|---|---|
| (*) nicht erfindungsgemäß | | | | | | |

**Rezepturbeispiel 19: (Zubereitung für Haarpflegeprodukte: Klares Conditioning-Shampoo)**

| | **1 (*)** | **2** | **3** | **4** | **5** | **6** |
|---|---|---|---|---|---|---|
| Natrium Laurylethersulfat | 10 | 9 | 3.5 | 3.5 | 0.5 | 8 |
| Natrium Myrethsulfat | - | - | 3.5 | 3.5 | 3.0 | 2 |
| Cocamidopropyl Betain | 4 | 4.5 | 3 | - | - | 3 |
| Natrium Cocoamphoacetat | - | - | - | 2.5 | - | 3 |
| Dinatrium PEG-5 Laurylcitrat Sulfosuccinat | - | - | - | - | 2.5 | 3 |
| Decylglucosid | - | - | - | 2 | 4.5 | - |
| Garcinia Cambogia Extract (Hydroxycitrat) | 1,00 | 1,00 | 1,00 | 1,00 | 1,00 | 1,00 |
| Carnitin | - | 1,00 | 1,00 | 1,00 | 1,00 | 1,00 |
| Verdicker | 0.1 | 0.2 | 0.3 | 0.4 | 0.5 | - |
| Polyquaternium-10 | 0.1 | 0.1 | 0.05 | 0.25 | 0.2 | 0,3 |
| Guar Hydroxypropyl-Trimonium Chlorid | - | 0.1 | - | - | 0.2 | - |
| Hydrolysiertes Seidenprotein | - | 0,1 | 0,2 | 0,3 | 0.3 | - |
| Iminodibersteinsäure | 0.1 | 0.1 | 0.2 | - | - | 0,5 |
| PEG-40 hydriertes Rizinusöl | 0.1 | 0.2 | 0.3 | 0.1 | 0.2 | 0,4 |
| Natrium Salicylat | - | - | 0.4 | - | | 0,2 |
| Natrium Benzoat | 0.5 | 0.5 | 0.4 | 0.4 | 0.4 | 0,4 |
| Benzophenon-4 | - | - | 0.1 | 0,2 | 0,3 | 0,4 |
| Zitronensäure | q.s. | q.s. | q.s. | q.s. | q.s. | q.s. |
| Parfüm | q.s. | q.s. | q.s. | q.s. | q.s. | q.s. |
| Wasser | ad 100 | ad 100 | ad 100 | ad 100 | ad 100 | ad 100 |

| | | | | | | |
|---|---|---|---|---|---|---|
| (*) nicht erfindungsgemäß | | | | | | |

**Rezepturbeispiel 20: (Zubereitung für Haarpflegeprodukte: Mildes Baby Shampoo)**

| | **1** | **2** | **3** | **4** | **5** | **6** |
|---|---|---|---|---|---|---|
| Natrium Myristylethersulfat | 4 | 4 | 5 | 5 | 4 | 4 |
| Decylglucosid | 4 | 4 | 4 | 4 | 4 | 2 |
| Dinatrium PEG-5 Laurylcitrat Sulfosuccinat | 4 | 4 | 3 | 5 | 5 | 3 |
| PEG-80 Sorbitan Laurat | 2 | 1 | 1 | - | 0.5 | - |
| Garcinia Cambogia Extract (Hydroxycitrat) | 1,00 | 1,00 | 1,00 | 1,00 | 1,00 | 1,00 |
| Carnitin | 2,00 | 2,00 | 2,00 | 2,00 | 2,00 | 2,00 |
| Verdicker | 0.1 | 0.1 | 0.1 | 0.2 | 0.3 | 0,4 |
| Polyquaternium-10 | 0.3 | 0.1 | 0.1 | 0.3 | - | 0,1 |
| Guar Hydroxypropyl-Trimonium Chlorid | - | - | 0.1 | 0.2 | 0.2 | - |
| Perlglanz | - | - | 4 | 2 | 2.5 | 1 |
| Trübungsmittel | - | - | - | - | 0.5 | 0,5 |
| Iminodibersteinsäure | - | 0.2 | 0.1 | 0.5 | 0.5 | 0,5 |
| PEG-40 hydriertes Rizinusöl | 0.2 | 0.3 | 0.4 | 0.2 | 0.3 | 0,4 |
| Natrium Salicylat | 0.2 | 0.2 | 0.2 | 0.4 | 0.4 | 0,4 |
| Natrium Benzoat | 0.4 | 0.4 | 0.4 | 0.4 | 0.4 | 0,4 |
| Natriumchlorid | 0.9 | 1.0 | 1.2 | 1,5 | 1,8 | - |
| Zitronensäure | q.s. | q.s. | q.s. | q.s. | q.s. | q.s. |
| Parfüm | q.s. | q.s. | q.s. | q.s. | q.s. | q.s. |
| Wasser | ad 100 | ad 100 | ad 100 | ad 100 | ad 100 | ad 100 |

**Rezepturbeispiel 21: (Zubereitung für Haarpflegeprodukte: Antischuppen Shampoo/Mildes Kopfhaut Shampoo)**

| | **1** | **2** | **3** | **4** | **5** | **6** |
|---|---|---|---|---|---|---|
| Natrium Laurylethersulfat | 9 | 9 | 9 | 9 | 10 | - |
| Natrium Myristylethersulfat | - | - | - | - | - | 6 |
| Cocamidopropyl Betain | 4 | 4 | 3 | 3 | 4 | 5 |
| Dinatrium PEG-5 Laurylcitrat Sulfosuccinat | 3 | 3 | 3 | 3 | - | - |
| Garcinia Cambogia Extract (Hydroxycitrat) | 1,00 | 1,00 | 1,00 | 1,00 | 1,00 | 1,00 |
| Carnitin | 1,00 | 1,00 | 1,00 | 1,00 | 1,00 | 1,00 |
| Natrium Cocoamphoacetat | - | - | - | - | - | 2.5 |
| Decylglucosid | - | - | - | - | - | 2.5 |
| Verdicker | 0.1 | 0.1 | 0.1 | 0.2 | 0.4 | 3 |
| Polyquaternium-10 | 0.3 | 0.1 | 0.1 | 0.3 | 0.1 | 0.3 |
| Guar Hydroxypropyl-Trimonium Chlorid | 0,1 | 0,1 | 0.2 | 0.2 | - | - |
| Climbazol | 0.5 | 0.5 | - | 0.5 | 1.0 | - |
| Piroctone Olamin | - | 0.5 | 0.3 | - | 0.5 | - |
| Laureth-9 | - | - | - | - | 2 | 2 |
| Panthenol | - | - | - | - | - | 0,1 |
| Harnstoff | | | | 3 | 4 | 5 |
| Perlglanz | 1.5 | 3 | 4 | 2 | 2.5 | - |
| Trübungsmittel | - | - | - | - | 0.5 | - |
| Iminodibersteinsäure | 0.1 | 0.2 | 0.1 | 0.5 | 0.5 | - |
| PEG-40 hydriertes Rizinusöl | 0.2 | 0.3 | 0.4 | 0.5 | 0.2 | - |
| Natrium Salicylat | 0.4 | 0.4 | 0.4 | 0.4 | 0.4 | 0.2 |
| Natrium Benzoat | 0.4 | 0.4 | 0.4 | 0.4 | 0.4 | 0.4 |
| Natriumchlorid | 0.9 | 1.0 | 1.2 | - | - | - |
| Zitronensäure | q.s. | q.s. | q.s. | q.s. | q.s. | - |
| Milchsäure | - | - | - | - | - | q.s. |
| Parfüm | q.s. | q.s. | q.s. | q.s. | q.s. | q.s |
| Wasser | ad 100 | ad 100 | ad 100 | ad 100 | ad 100 | ad 100 |

## Patentansprüche

1. Nichttherapeutische Verwendung von topischen Zubereitungen enthaltend Hydroxycitrat zur Verringerung der Sebumproduktion in der Talgdrüse **dadurch gekennzeichnet, dass** als Hydroxycitrat enthaltende Komponente ein *Garcinia Cambogia-*Extrakt verwendet wird und zusätzlich Carnitin in der topischen Zubereitung enthalten ist und die Zubereitung einen Hydroxycitrat-Anteil von 0,25% bis 2% und einen Carnitin-Anteil von 1% bis 4% aufweist.

2. Verwendung nach Patentanspruch 1, **dadurch gekennzeichnet, dass** zusätzlich saure Aluminium- oder Alumuminium-Zirkoniumsalze in der topischen Zubereitung enthalten sind.

3. Verwendung nach einem der vorangehenden Patentansprüche, **dadurch gekennzeichnet, dass** zusätzlich ein vernetzter Verdicker in der topischen Zubereitung enthalten ist.

4. Verwendung nach Patentanspruch 3, **dadurch gekennzeichnet, dass** der vernetzte Verdicker Ammonium Acryloyldimethyltaurate/VP Crosspolymer oder Acrylates/C10-30 Alkyl Acrylate Crosspolymer ist.

5. Verwendung nach einem der vorangehenden Patentansprüche, **dadurch gekennzeichnet, dass** zusätzlich ein nicht vernetzter Verdicker der topischen Zubereitung enthalten ist.

6. Verwendung nach Patentanspruch 5, **dadurch gekennzeichnet, dass** der nicht vernetzte Verdicker Xanthan Gum, Carrageenan oder ein Polymer, das überwiegend aus Acrylsäureeinheiten aufgebaut ist, darstellt.

## Claims

1. Non-therapeutic use of topical preparations comprising hydroxycitrate for reducing sebum production in the sebaceous gland, **characterized in that** the hydroxycitrate-containing component used is a *Garcinia Cambogia* extract, and additional carnitine is present in the topical preparation, and the preparation has a hydroxycitrate fraction of from 0.25% to 2% and a carnitine fraction of from 1% to 4%.

2. Use according to Patent Claim 1, **characterized in that** acidic aluminium salts or aluminium-zirconium salts are present in the topical preparation.

3. Use according to one of the preceding patent claims, **characterized in that** a crosslinked thickener is additionally present in the topical preparation.

4. Use according to Patent Claim 3, **characterized in that** the crosslinked thickener is Ammonium Acryloyldimethyltaurate/VP Crosspolymer or Acrylates/C10-30 Alkyl Acrylate Crosspolymer.

5. Use according to one of the preceding patent claims, **characterized in that** a non-crosslinked thickener is additionally present in the topical preparation.

6. Use according to Patent Claim 5, **characterized in that** the non-crosslinked thickener is xanthan gum, carrageenan or a polymer which is predominantly composed of acrylic acid units.

## Revendications

1. Utilisation non thérapeutique de compositions topiques, contenant de l'hydroxycitrate pour diminuer la production de sébum dans les glandes sébacées, **caractérisée en ce qu'**on utilise comme composant contenant de l'hydroxycitrate un extrait de Garcinia Cambogia et la composition topique contient en outre de la carnitine et la composition présente une proportion d'hydroxycitrate de 0,25% à 2% et une proportion de carnitine de 1% à 4%.

2. Utilisation selon la revendication 1, **caractérisée en ce que** la composition topique contient en outre des sels acides d'aluminium ou d'aluminium-zirconium.

3. Utilisation selon l'une quelconque des revendications précédentes, **caractérisée en ce que** la composition topique contient en outre un épaississant réticulé.

4. Utilisation selon la revendication 3, **caractérisée en ce que** l'épaississant réticulé est le polymère réticulé "Ammonium Acryloyldimethyltaurate/VP Crosspolymer" ou le polymère réticulé "Acrylates/C10-30 Alkyle Acrylate Crosspolymer".

5. Utilisation selon l'une quelconque des revendications précédentes, **caractérisée en ce que** la composition topique contient en outre un épaississant non réticulé.

6. Utilisation selon la revendication 5, **caractérisée en ce que** l'épaississant non réticulé est la gomme de xanthane, le carraghénane ou un polymère qui est principalement constitué d'unités d'acide acrylique.
